(19) Europäisches Patentamt
European Patent Office
Office européen des brevets

(11) Numéro de publication: **0 061 393**
**B1**

# (12) FASCICULE DE BREVET EUROPEEN

(45) Date de publication du fascicule du brevet: **31.10.84**

(51) Int. Cl.³: **C 07 D 301/14**, C 07 D 303/04

(21) Numéro de dépôt: **82400477.4**

(22) Date de dépôt: **16.03.82**

## (54) Procédé continu de préparation de l'oxyde de propylène.

(30) Priorité: **24.03.81 FR 8105811**

(43) Date de publication de la demande: **29.09.82 Bulletin 82/39**

(45) Mention de la délivrance du brevet: **31.10.84 Bulletin 84/44**

(84) Etats contractants désignés: **BE CH DE FR GB IT LI NL**

(56) Documents cités:
**EP-A-0 025 381**
**FR-A-2 300 085**
**FR-A-2 369 274**
**GB-A-1 497 970**

(73) Titulaire: **ATOCHEM, 12/16, allée des Vosges, F-92400 Courbevoie (FR)**

(72) Inventeur: **Lecoq, Jean-Claude, 10 A, rue Josserand, F-69630 Chaponost (FR)**
Inventeur: **Pralus, Michèle, 3, rue des Gosses, F-69450 Saint-Cyr au Mont d'Or (FR)**
Inventeur: **Schirmann, Jean-Pierre, 49, chemin de la Glacière, F-69600 Oullins (FR)**

(74) Mandataire: **Rochet, Michel et al, ATOCHEM Département Propriété Industrielle Cédex 22, F-92091 Paris la Défense (FR)**

**EP 0 061 393 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne un procédé continu de préparation de l'oxyde de propylène, à partir de propylène et de peroxyde d'hydrogène, utilisant comme intermédiaire une solution organique brute d'acide perpropionique obtenue par réaction du peroxyde d'hydrogène sur l'acide propionique en présence d'un catalyseur acide fort.

Les réactions bien connues mises en jeu dans ce procédé sont les suivantes:

– réaction du peroxyde d'hydrogène avec l'acide propionique pour former l'acide perpropionique:

$$H_2O_2 + CH_3—CH_2—COOH \rightleftharpoons CH_3—CH_2—COOOH + H_2O \qquad (1)$$

– réaction de l'acide perpropionique avec le propylène pour former l'oxyde de propylène:

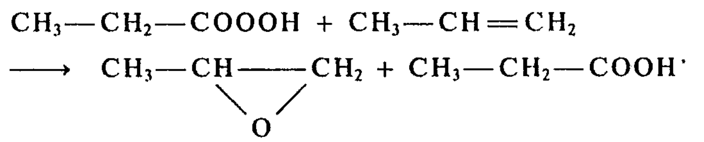

(2)

La réaction (1) est une réaction équilibrée et lente. En raison de l'instabilité des acides percarboxyliques, cette réaction est généralement conduite à température modérée, et dans ces conditions l'état d'équilibre n'est atteint qu'au bout de plusieurs heures. Une telle durée n'est pas admissible pour un procédé industriel et l'on doit avoir recours à un catalyseur.

Les catalyseurs acide fort proposés et utilisés jusqu'à maintenant sont les acides minéraux forts, tels que l'acide phosphorique, l'acide sulfurique et l'acide chlorhydrique, ou les acides organiques forts, tels que les acides alkylou arylsulfoniques, ou encore l'acide trifluoracétique. Ces acides forts sont utilisés à raison de un à quelques pour cent en poids du mélange réactionnel. On a aussi recommandé d'utiliser comme catalyseur des résines échangeuses de cations à fonction acide fort, telles que les résines connues dans le commerce sous les marques déposées »DOWEX 50« ou »AMBERLITE IR-120«, et qui sont des acides polystyrènesulfoniques réticulés.

Le peroxyde d'hydrogène est le plus souvent mis en oeuvre sous forme de solutions aqueuses commerciales contenant de 30 à 70% d'eau. Comme la réaction (1) fournit aussi une molécule d'eau par molécule d'acide perpropionique, il est clair que l'état d'équilibre est atteint bien avant que le peroxyde d'hydrogène soit totalement transformé. Dans ces conditions le produit de la réaction est en fait un mélange d'acide propionique, de peroxyde d'hydrogène, d'acide perpropionique, d'eau et de catalyseur acide.

Les solutions aqueuses d'acide percarboxylique que l'on obtient par exemple en suivant les indications des demandes de brevets de la République Fédérale d'Allemagne n° 1 165 576 et 1 170 926 ne sont pas utilisables directement comme réactifs pour l'époxydation des $\alpha$-oléfines. En effet la présence simultanée dans ces solutions d'eau, d'acide carboxylique et de catalyseur acide fort, favorise les réactions d'ouverture des cycles oxiranes formés au cours de l'époxydation des oléfines et conduit à la formation de produits indésirables, comme les glycols ou leurs monoesters ou diesters. Ce point est largement développé par exemple dans la demande de brevet n° 2 519 296 de la République Fédérale d'Allemagne, relative à l'obtention de solutions organiques d'acides percarboxyliques, exemptes d'eau et de peroxyde d'hydrogène. Il est certes possible d'effectuer une neutralisation du catalyseur acide fort à l'aide de réactifs basiques, mais les sels ainsi formés sont eux-mêmes susceptibles d'engendrer des réactions secondaires parasites ou pour le moins posent de délicats problèmes de séparation.

Pour pallier ces inconvénients, différents procédés ont été proposés pour préparer des solutions organiques d'acides percarboxyliques anhydres ou ayant une très faible teneur en eau.

Une première méthode consiste à préparer le peracide par oxydation d'un aldéhyde dans un solvant organique. Le brevet n° 1 043 315 de la République Fédérale d'Allemagne décrit par exemple la préparation d'une solution d'acide peracétique à partir d'acétaldéhyde dans le l'acétate d'éthyle. Mais il est alors indispensable de pouvoir valoriser l'acide acétique formé comme sous-produit.

Un autre procédé consiste à préparer d'abord une solution non-aqueuse de peroxyde d'hydrogène, pour la faire réagir ensuite sur un acide carboxylique. Mais la solution de peracide obtenue, comme par exemple dans la demande de brevet n° 2 038 318 de la République Fédérale d'Allemagne, doit malgré tout être déshydratée par distillation azéotropique sous pression réduite.

D'autres procédés d'obtention de solutions non-aqueuses d'acides percarboxyliques reposent sur le principe d'extraction par un solvant organique, tel que le benzène ou le dichloropropane, d'une solution aqueuse contenant l'acide percarboxylique, l'acide carboxylique, le peroxyde d'hydrogène non transformé et le catalyseur acide fort. De tels procédés sont décrits par exemple dans les demandes de brevets n° 2 141 156 et 2 262 970, publiées en République Fédérale d'Allemagne.

Il a aussi été proposé d'effectuer la synthèse du peracide simultanément à l'extraction par le solvant, en effectuant l'opération par circulation à contre-courant dans une colonne d'extraction liquide-liquide. C'est la technique décrite dans les brevets français n° 2 300 085 et 2 369 275 qui ne permet

toutefois pas d'atteindre une peroxydation complète malgré l'emploi d'une quantité massique d'acide sulfurique.

Dans tous les cas cependant, la phase organique, séparée après décantation, contient encore un certain nombre d'impuretés (eau, catalyseur, peroxyde d'hydrogène non transformé), ce qui oblige à prévoir une étape de purification avant l'utilisation dans des réactions d'oxydation ou d'époxydation.

Cette purification peut s'effecteur par distillation, comme préconisé dans la demande de brevet allemand n°2 519 296, où l'on élimine l'eau et le peroxyde d'hydrogène résiduels par distillation azéotropique sous pression réduite, ou par des lavages au moyen de solutions aqueuses, comme proposé dans la demande de brevet européen n°18 692 et dans la demande de brevet n°2 519 290 de la République Fédérale d'Allemagne.

D'autre part, le phase aqueuse séparée après décantation contient également un certain nombre de produits (acide percarboxylique, acide carboxylique, solvant organique, peroxyde d'hydrogène et catalyseur) dont on peut diminuer ou augmenter les concentrations respectives au moyen de recyclage ou de distillations, comme il est décrit dans les demandes de brevets n° 2 519 287, 2 519 288, 2 519 289, 2 519 293, 2 519 294, 2 519 295 et 2 519 301 de la République Fédérale d'Allemagne. Ces opérations, dont l'objectif principal est la récupération du peroxyde d'hydrogène non transformé, nécessitent un appareillage complexe, réalise en matériaux coûteux, tels que le zirconium, le tantale ou leurs alliages. De tels procédés sont incompatibles avec une production industrielle économique de l'oxyde de propylène.

La possibilité de déplacer vers la droite l'équilibre de la réaction (1) par élimination, de l'eau, au fur et à mesure de l'avancement de la réaction, à l'aide d'un entraîneur azéotropique est également bien connue. C'est ainsi que le brevet des Etats-Unis n°2 814 641 enseigne d'opérer la réaction en présence d'un solvant inerte, possédant un point d'ébullition à la pression atmosphérique compris entre 50 et 130°C et formant avec l'eau un hétéro-azéotrope. On élimine ainsi complètement l'eau de dilution des réactifs et au moins une partie de l'eau formée au cours de la réaction. Cette opération s'effectue de préférence sous pression réduite, à des températures comprises entre 30 et 65°C, car les températures supérieures favorisent la décomposition du peroxyde d'hydrogène et/ou de peracide. Cela conduit à des temps de réaction de l'ordre de 8 à 10 heures, incompatibles avec un procédé industriel de préparation de l'oxyde de propylène. D'autre part, la quantité de catalyseur acide fort utilisée, soit au moins 0,2 partie en poids, et de préférence 1 à 2 parties en poids pour 100 parties d'acide carboxylique (utilisé au moins en quantité stoechiométrique par rapport au peroxyde d'hydrogène), est telle qu'il est nécessaire de prévoir l'élimination de ce catalyseur avant de pouvoir utiliser le peracide dans une réaction telle que l'époxydation du propylène.

Il a aussi été proposé dans le brevet de Etats-Unis n° 2 877 266, utilisant la même technique et le dichloro-1,2-éthane comme entraîneur azéotropique, de pousser la réaction jusqu'à un degré d'achèvement supérieur, sans utiliser d'excès important de réactifs. La réaction est normalement effectuée en présence de petites quantités, non précisées, d'un catalyseur convenable tel que l'acide sulfurique. La distillation de l-hétéro-azéotrope est effectuée sous pression réduite (140 à 175 mm de mercure, soit 18,6 à 23,3 kPa).

On connaît un autre procéde, décrit dans le brevet des Etats-Unis n° 3 284 491, selon lequel on travaille en présence de deux solvants, l'un formant l'hétéro-azéotrope et l'autre servant de solvant intermédiaire entre le premier et l'eau. On élimine ainsi les risques potentiels d'explosion du procédé précédent, en particulier en supprimant toute possibilité de démixtion du système réactionnel en deux phases (risque de former une phase aqueuse où le peroxyde d'hydrogène et/ou l'acide percarboxylique atteignent une concentration suffisamment élevée pour provoquer une éventuelle explosion). Toutefois ce procédé est difficile à mettre en oeuvre économiquement, parce que le deuxième solvant, entièrement miscible à l'eau, se retrouve à la fois dans la solution finale de peracide, où il peut subir lui-même des réactions d'oxydation (cas du dioxanne par exemple) et dans le dispositif de séparation de l'hétéro-azéotrope formé avec l'eau et le premier solvant, où il perturbe la séparation de ces deux substances. L'eau décantée dans le dispositif de séparation contient des quantités non négligeables de ce deuxième solvant, qu'il faut récupérer.

Dans la demande de brevet européen n° 4 407 il a été préconisé au contraire de maintenir dans le mélange réactionnel une quantité d'eau suffisante pour qu'il se forme une phase aqueuse distincte de la phase organique contenant le solvant du peracide capable de former avec l'eau un hétéro-azéotrope. Cette façon d'opérer est motivée par le fait que la réaction de formation du peracide, qui a lieu essentiellement dans la phase aqueuse, est ainsi notablement accélérée par rapport aux procédés où la teneur en eau diminue dans le milieu au fur et à mesure de l'entraînement azéotropique. Cependant la nécessité de maintenir une émulsion sous pression réduite et la nécessité de contrôler en permanence la proportion totale de composés peroxydés dans le mélange réactionnel, pour diminuer les risques d'explosion, rendent ce procédé difficile à mettre en oeuvre et potentiellement dangereux dans le cas de productions industrielles à fort tonnage.

Il a été également proposé, dans le brevet britannique n° 931 119, d'utiliser un ester d'acide carboxylique à la fois comme réactif et comme entraîneur azéotropique de l'eau. Mais les conditions opératoires de température (40 à 50°C) et de pression (60 à 120 mm de mercure, soit 8 à 16 kPa) conduisent à des temps de réaction très longs et/ou à des conversions faibles du peroxyde d'hydrogène.

D'autre part, comme il est souligné dans la demande de brevet n° 1 962 671 de la République Fédérale d'Allemagne, la réaction de perhydrolyse de l'ester, qui se produit lorsqu'on met en oeuvre un tel procédé, libère une molécule d'alcool suspectible d'induire des réactions secondaires nuisibles dans les réactions d'oxydation ultérieures.

Il a été enfin proposé dans le brevet européen n° 0 025 381 d'effectuer la réaction du peroxyde d'hydrogène sur des acides carboxyliques solubles dans l'eau, en présence d'un acide borique comme catalyseur, avec élimination de l'eau au moyen d'un entraîneur azéotropique. Mais le processus de formation de l'acide percarboxylique en présence d'un acide borique est toutefois de nature fondalement différente de celle du processus de formation de l'acide percarboxylique en présence d'un catalyseur acide fort.

La réaction d'époxydation des oléfines par les peracides carboxyliques a été décrite dès 1909 par PRILESCHAJEW (Berichte, 42, 4811, 1909). L'obtention d'oxyde de propylène par réaction de l'acide peracétique sur le propylène a été signalée en 1935 par J. STUURMAN (Proc. Acad. Sci. Amsterdam, 38, 450—452, 1935). Un procédé de fabrication d'oxyde de propylène est d'autre part décrit dans le brevet britannique n° 900 836; ce procédé utilise une solution d'acide peracétique dans un mélange d'acide acétique et d'acétone obtenu par oxydation en phase vapeur de l'acétaldéhyde. En dehors du fait qu'un tel procédé nécessite, comme déjà souligné précedemment, une valorisation de l'acide acétique co-produit, les rendements en oxyde de propylène ne sont pas quantitatifs (86,3%de peracide transformé selon l'exemple 4 de ce brevet). D'autre part une partie non négligeable du propylène est transformée en propylèneglycol et en monoacétate du propylèneglycol. Cette perte en rendement constitue l'obstacle principal des procédés de préparation de l'oxyde de propylène au moyen d'un acide percarboxylique, comme l'explique la demande de brevet n° 2 633 395 de la République Fédérale d'Allemagne.

Poursuivant ses travaux dans le domaine de l'utilisation du peroxyde d'hydrogène en chimie organique, la demanderesse vient de découvrir de manière tout-à-fait inattendue qu'il est possible d'époxyder le propylène, avec des rendements pratiquement quantitatifs en oxyde de propylène et un taux de transformation pratiquement négligeable en glycols et en esters, en utilisant directement, sans aucun traitement préalable, des solutions organiques brutes d'acide perpropionique, sensiblement anhydres, obtenues par réaction de solutions aqueuses de peroxyde d'hydrogène sur l'acide propionique en présence de très faibles quantités d'un catalyseur acide fort, avec élimination de l'eau par entraînement azéotropique à la pression atmosphérique et à des températures comprises entre 90 et 100°C.

Il n'était pas du tout évident pour l'homme du métier, au vu de l'art antérieur, que l'utilisation de quantités très réduites de catalyseur acide fort permettrait d'effectuer rapidement la réaction de formation de l'acide perpropionique à des températures relativement élevées, sans qu'il y ait décomposition du peroxyde d'hydrogène et/ou du peracide, et que les solutions organiques brutes d'acide perpropionique ainsi obtenues pourraient être utilisées directement dans l'époxydation du propylène, sans qu'il soit nécessaire de neutraliser le catalyseur ou d'effectuer un quelconque traitement de purification.

Le procédé selon l'invention est réalisé en trois étapes, menées en continu. Dans une première étape, on prépare une solution sensiblement anhydre d'acide perpropionique en mettant en contact l'acide propionique, l'entraîneur azéotropique, le peroxyde d'hydrogène et le catalyseur, à une température comprise entre 90 et 100°C et en éliminant continuellement l'eau à la pression atmosphérique.

L'entraîneur azéotropique est choisi avantageusement parmi les solvants organiques de point d'ébullition inférieur à 100°C sous la pression atmosphérique normale, et capables de former avec l'eau un hétéro-azéotrope à point d'ébullition minimum. On peut citer, à titre d'exemples non limitatifs des entraîneurs utilisables, les solvants chlorés aliphatiques, tels que le chloroforme, le tétrachlorure de carbone, le chlorure de méthylène, le dichloro-1,2-éthane, le dichloro-1,2-propane, et les solvants hydrocarbonés tels que le cyclohexane et le benzène.

La quantité de solvant entraîneur azéotropique est comprise entre 50 et 75% en poids du mélange réactionnel. Elle est choise de telle sorte que l'on puisse régler à volonté le point d'ébullition du mélange dans une zone de température comprise entre 90 et 100°C à la pression atmosphérique normale, et éliminer de façon efficace et rapide l'eau du milieu.

Le peroxyde d'hydrogène peut être mis en oeuvre dans le cadre de l'invention sous forme de solution organique anhydre, mais il est préféré d'utiliser les solutions aqueuses commerciales, titrant de 30 à 75% en poids de peroxyde d'hydrogène.

Le rapport molaire du peroxyde d'hydrogène à l'acide propionique est compris entre 0,2 et 1, et de préference entre 0,3 et 0,5.

Le catalyseur acide fort utilisé est soit un acide fort minéral, tel que l'acide sulfurique ou l'acide phosphorique, soit un acide fort organique, tel qu'un acide alcanesulfonique, un acide arylsulfonique ou un acide carboxylique ou sulfonique perfluoré. L'acide sulfurique est le catalyseur préféré.

Ce catalyseur est utilisé à raison de seulement 0,0005 à 0,002 mole par mole de peroxyde d'hydrogène, ce qui constitue l'originalité principale du procédé selon l'invention.

On peut avantageusement introduire dans le milieu réactionnel un inhibiteur de réactions radicalaires, tel que l'hydroquinone, l'acide dipicolinique, ou tout autre stabilisant connu pour éviter la décomposition du peracide formé.

Il peut être aussi avantageux d'ajouter au mélange réactionnel des stabilisants du peroxyde d'hydrogène, tels que les polyphosphates ou les dérivés de l'acide éthylènediaminetétracétique.

Cette première étape de réaction peut s'effectuer dans les réacteurs les plus divers, mais on préfère utiliser des réacteurs permettant la distillation en cours de réaction de l'hétéro-azéotrope avec l'eau. Il est particulièrement avantageux d'utiliser une batterie de réacteurs en cascade, reliés chacun à une colonne de distillation à plateaux ou à garnissage. Il est aussi possible de réunir les phases vapeurs des réacteurs et de les envoyer dans une unique colonne de distillation. Dans ce cas le reflux de solvant entraîneur azéotropique peut être renvoyé dans un seul réacteur ou dans chacun des réacteurs en série.

La séparation par décantation de l'azéotrope eau — solvant organique recueilli en tête de colonne peut être réalisée selon diverses techniques connues en elles-mêmes, telles que la décantation par gravité ou le passage à travers un garnissage coalescent. Le solvant organique décanté peut être recyclé tel quel dans la colonne de distillation en vue d'assurer un reflux suffisamment important pour éviter de distiller de l'acide propionique, de l'acide perpropionique et du peroxyde d'hydrogène. Il peut également être séché préalablement par toute technique connue en elle-même, telle qu'un passage sur un garnissage déshydratant. Avantageusement, la colonne de distillation azéotropique reliée au réacteur de formation du peracide peut comprendre un tronçon d'épuisement, assurant la déshydratation parfaite du solvant entraîneur.

La solution organique brute ainsi obtenue contient, à côté de l'acide perpropionique, de l'acide propionique non transforme, le catalyseur acide, une petite quantité de peroxyde d'hydrogène, pouvant aller jusqu'à 1% en poids et une quantite d'eau inférieure à 1% en poids.

Dans la deuxième étape du procédé, cette solution organique brute d'acide perpropionique est mise en présence d'un excès de propylène dans un réacteur maintenu à une température comprise entre 40 et 80°C et sous une pression comprise entre 2 et 20 bars. La pression de réaction est choisie de telle sorte que le milieu réactionnel soit maintenu en phase liquide.

Le rapport molaire du propylène à l'acide perpropionique est compris entre 1,01 et 10. Il se situe de préférence entre 2 et 6.

Les réacteurs utilisés pour effectuer la réaction d'epoxydation du propylène sont des réacteurs favorisant les échanges thermiques, de manière à bien contrôler la température de réaction. On peut utiliser par exemple des reacteurs pistons tubulaires ou des autoclaves agités, montes de préférence en cascade.

Dans la troisième étape du procédé, le traitement du mélange réactionnel s'effectue de façon connue en soi. On sépare successivement par distillation le propylène non transformé, l'oxyde de propylène que l'on peut purifier par des phases d'étêtage et/ou d'équeutage, puis le solvant organique et l'acide propionique, qui peuvent être recyclés directement à la première étape de synthèse du peracide.

Le procédé selon l'invention peut par exemple être réalisé dans un appareillage tel que celui représenté schématiquement dans la figure en annexe.

Dans une batterie de réacteurs en cascade 1, reliés chacun à une colonne de distillation 2, on introduit par la voie 3 une solution aqueuse de peroxyde d'hydrogène à 70% en poids, contenant le catalyseur acide fort et le stabilisant du peracide; par la voie 4 on introduit une solution d'acide propionique dans le solvant organique, constituée d'une part des produits 18 de recyclage, issus de la colonne de distillation 17, et d'autre part d'un appoint 5 de produit frais pour compenser les faibles pertes. Les vapeurs constituant la phase gazeuse du réacteur 1 sont envoyées dans la colonne de distillation 2, d'où l'on sépare par décantation en tête de colonne la phase aqueuse de l'hétéro-azéotrope condensé, tandis que la phase organique assure le reflux nécessaire à la bonne séparation de l'azéotrope. Si nécessaire un tronçon d'épuisement en pied de colonne peut permettre de renvoyer au réacteur par la voie 8 une phase organique liquide parfaitement anhydre. L'eau formée au cours de la réaction et celle apportée par les réactifs sont éliminées par la voie 7.

La solution organique brute d'acide perpropionique 9 est envoyée dans un réacteur tubulaire 10, tandis qu'un excès de propylène est injecté en phase liquide par la voie 11. Le propylène injecté est constitué d'une partie recyclée, issue de la colonne 14 par la voie 15 et d'un appoint de propylène frais 12. La solution sortant du réacteur est envoyée par la voie 13 dans une colonne de »stripping« 14, où l'on récupère en tête le propylène en excès. A la base de cette colonne on récupère par la voie 16 une solution d'oxyde de propylène, qui est purifiée dans la colonne 17. On récupère en pied de cette colonne le mélange d'acide propionique et de solvant organique, qui est renvoyé au réacteur 1 par la voie 18. Une purge de produits lourds est effectuée si nécessaire par la voie 19. L'oxyde de propylène récupéré en tête de colonne par la voie 20 est purifié ultérieurement si cela s'avère nécessaire.

L'exemple suivant, donné à titre non limitatif, illustre la mise en oeuvre pratique de l'invention.

## Exemple

Dans une batterie de deux réacteurs en verre de 250 $cm^3$, montés en cascade et équipés chacun d'une colonne de distillation OLDERSHAW de 10 plateaux surmontée d'un condenseur à reflux, on introduit en continu 250 g/h d'une solution d'acide propionique à 50% en poids dans le dichloro-1,2-éthane, et 40,3 g/h d'une solution aqueuse à 70,7% en poids de peroxyde d'hydrogène (soit 0,838 mole/heure),

contenant également 0,33% en poids d'acide sulfurique et 0,5% en poids d'acide dipicolinique. La température dans chaque réacteur est respectivement de 90°C et de 94°C.

En tête de chaque colonne de distillation, où l'on observe respectivement des températures de 75°C et de 76°C, on soutire, après condensation à 20°C de l'hétéro-azéotrope eaudichloro-1,2-éthane et décantation, une phase aqueuse que l'on rassemble pour évacuation. On élimine ainsi au total 38,7 g/h d'une phase aqueuse contenant 0,7% en poids de peroxyde d'hydrogène (soit 0,008 mole/heure). La phase organique condensée est refluée dans la colonne.

On soutire en continu du second réacteur 281 g/h d'une solution organique contenant 25,1% en poids d'acide perpropionique (0,784 mole/heure) et 0,3% en poids de peroxyde d'hydrogène (0,026 mole/heure).

A ce stade, le rendement en acide perpropionique, rapporté au peroxyde d'hydrogène frais introduit dans le cycle, est de 93,5%. Par rapport au peroxyde d'hydrogène transformé, le rendement en acide perpropionique est de 97,5%. La solution organique brute d'acide perpropionique est injectée en continu, à raison de 281 g/h, en même temps que 98,3 g/h de propylène (soit 2,34 mole/heure), dans un réacteur tubulaire du type piston, de 2 m de hauteur et de 10 mm de diamètre, maintenu à 50°C. La pression dans le réacteur est de 10 bars.

A la sortie du réacteur, le mélange réactionnel est envoyé dans une colonne de »stripping« pour récupérer en tête le propylène non transformé. La phase liquide recueillie en continu au pied de la colonne, au débit de 350 g/h, contient 13,2% d'oxyde de propylène. Rapporté à l'oxygène peroxydique utilisé dans la deuxième étape, le rendement en oxyde de propylène est de 98,3%. Rapporté au peroxyde d'hydrogène frais introduit dans la première étape, ce rendement est de 95,02%.

## Revendications

1. Procédé continu de préparation de l'oxyde de propylène, comprenant la préparation d'une solution organique d'acide percarboxylique par réaction du peroxyde d'hydrogène sur un acide carboxylique en présence d'un solvant entraîner azéotropique de l'eau, et l'époxydation du propylène par cette solution d'acide percarboxylique, caractérisé en ce que:

a) la solution organique d'acide percarboxylique, contenant moins de 1% en poids d'eau et jusqu'a 1% en poids de peroxyde d'hydrogène, est obtenue en faisant réagir entre 90 et 100°C une solution d'acide propionique dans un solvent organique, inerte vis-à-vis du peracide et capable de former avec l'eau un hétéro-azéotrope à point d'ébullition inférieur à 100°C sous pression atmosphérique, avec une solution aqueuse titrant de 30 à 75% en poids de peroxyde d'hydrogène, en présence de 0,0005 à 0,002 mole d'un catalyseur acide fort par mole de peroxyde d'hydrogène et d'un inhibiteur de réactions radicalaires, et en éliminant en continu l'eau par distillation azéotropique, les proportions de réactifs étant choisies de manière à ce que le rapport molaire du peroxyde d'hydrogène à l'acide propionique soit compris entre 0,2 et 1 et à ce que le solvant entraîneur azéotropique représente de 50 à 75% en poids du mélange réactionnel;

b) la solution organique brute d'acide perpropionique obtenue en pied de la distillation azéotropique de l'étape (a) est mise en réaction avec un excés de propylène, de manière à ce que le rapport molaire du propylène à l'acide perpropionique soit compris entre 1,01 et 10, à une température comprise entre 40 et 80°C, sous une pression comprise entre 2 et 20 bar, choisie de telle sorte que le milieu réactionnel soit maintenu en phase liquide;

c) le mélange réactionnel issu de l'étape (b) est traité de façon connue en soi pour séparer successivement le propylène non transformé, qui est recyclé à l'étape (b), l'oxyde de propylène, qui est extrait, le solvant organique et l'acide propionique, qui sont recyclés ensemble à l'étape (a) de synthèse du peracide.

2. Procédé selon la revendication 1, caractérisé en ce que le mélange réactionnel contient un stabilisant du peroxyde d'hydrogène.

## Patentansprüche

1. Kontinuierliches Verfahren zur Herstellung von Propylenoxid, das die Herstellung einer organischen Lösung von Percarbonsäure durch Umsetzung von Wasserstoffperoxid mit einer Carbonsäure in Anwesenheit eines Lösungsmittels als azeotropem Schleppmittel für Wasser, und die Epoxidierung von Propylen durch diese Percarbonsäurelösung umfaßt, dadurch gekennzeichnet, daß:

a) die organische Percarbonsäurelösung mit weniger als 1 Gew.% Wasser und bis 1 Gew.% Wasserstoffperoxid dadurch erhalten wird, daß man eine Lösung aus Propionsäure in einem organischen Lösungsmittel, das gegenüber der Persäure inert ist und zusammen mit dem Wasser ein heteroazeotropes Gemisch mit einem Siedepunkt unter 100°C bei Atmosphärendruck bilden kann, mit einer wäßrigen 30 bis 75 gew.%igen Lösung von Wasserstoffperoxid, in Gegenwart von 0,0005 bis 0,002 Mol eines stark sauren Katalysators pro Mol Wasserstoffperoxid und in Gegenwart eines Radikalreaktionen-Inhibitors bei Temperaturen zwischen 90 und 100°C umsetzt und das Wasser durch azeotrope Destillation kontinuierlich entfernt, wobei die Mengen der Reaktanden so gewählt werden, daß das Molverhältnis zwischen Wasserstoffperoxid und Propionsäure 0,2 bis 1 beträgt und das als azeotropes Schleppmittel wirkende Lösungsmittel 50 bis 75 Gew.% des Reaktionsgemisches ausmacht;

b) die rohe organische Perpropionsäurelösung, die als Sumpf der azeotropen Destillation gemäß Stufe (a) erhalten wird, bei einer Temperatur zwischen 40 und 80°C und unter einem Druck zwischen 2 und 20 bar, der so gewählt ist, daß das Reaktionsgemisch in der flüssigen Phase bleibt, mit einem derartigen Überschuß an Propylen umgesetzt wird, daß das Molverhältnis zwischen Propylen und Perpropionsäure zwischen 1,01 und 10 beträgt;

c) das Reaktionsgemisch aus der Stufe (b) auf eine an sich bekannte Weise behandelt wird, um nacheinander das nicht umgesetzte Propylen, das in die Stufe (b) zurückgeführt wird, das Propylenoxid, das abgezogen wird, und das organische Lösungsmittel und die Propionsäure abzutrennen, die zusammen in die Synthesestufe der Persäure (a) zurückgeführt werden.

2. Verfahren nach Anspruch 1, dadurch gekennzeichnet, daß das Reaktionsgemisch ein Stabilisierungsmittel für das Wasserstoffperoxid enthält.

## Claims

1. Continuous process für the preparation of propylene oxide, which comprises the preparation of an organic solution of percarboxylic acid by reacting hydrogen peroxide with a carboxylic acid in the presence of a solvent which azeotropically entrains water, and the epoxidation of propylene by this percarboxylic acid solution, characterised in that:

a) the organic solution of percarboxylic acid, containing less than 1% by weight of water and up to 1% by weight of hydrogen peroxide, is obtained by reacting, a between 90 and 100°C, a solution of propionic acid in an organic solvent which is inert towards the peracid and capable of forming, with water, a hetero-azeotrope of boiling point below 100°C under atmospheric pressure, with an aqueous solution containing from 30 to 75% by weight of hydrogen peroxide, in the presence of 0.0005 to 0.002 mole of a strongly acid catalyst per mole of hydrogen peroxide and of an inhibitor of radical reactions, and continuously removing the water by azeotropic distillation, the proportions of the reactants being so chosen that the molar ratio of hydrogen peroxide to propionic acid is between 0.2 and 1 and that the azeotropically entraining solvent represents from 50 to 75% by weight of the reaction mixture;

b) the crude organic solution of perpropionic acid which is obtained as the bottom product from the azeotropic distillation of stage (a) is reacted with an excess of propylene, so that the molar ratio of propylene to perpropionic acid is between 1.01 and 10, at a temperature of between 40 and 80°C, under a pressure of between 2 and 20 bar, the pressure being so chosen that the reaction medium is kept in the liquid phase; and

c) the reaction mixture issuing from stage (b) is treated in a manner known per se so as to separate off successively unconerted propylene, which is recycled to stage (b), the propylene oxide, which is extracted, the organic solvent and the propionic acid, which are jointly recycled to the peracid synthesis stage (a).

2. Process according to Claim 1, characterised in that the reaction mixture contains a stabiliser for hydrogen peroxide.

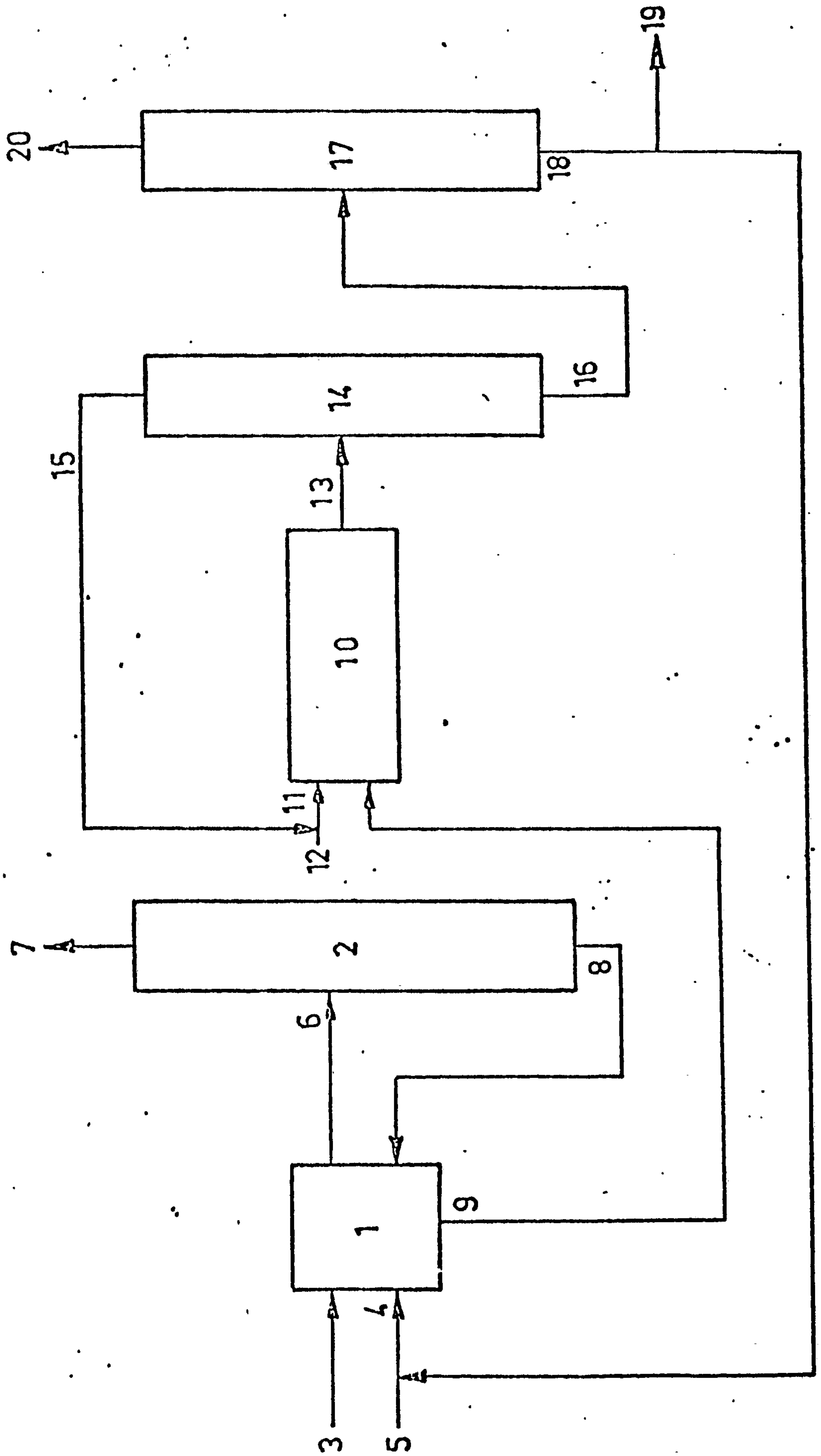
1
2
3
4
5
6
7
8
9
10
11
12
13
14
15
16
17
18
19
20